(19) Europäisches Patentamt
European Patent Office
Office européen des brevets

(11) Publication number: **0 004 173**
**B1**

(12) # EUROPEAN PATENT SPECIFICATION

(45) Date of publication of patent specification: **22.09.82**

(21) Application number: **79300332.8**

(22) Date of filing: **06.03.79**

(51) Int. Cl.³: **C 07 D 487/04,**
A 61 K 31/505
//C07D239/30, (C07D487/04,
239/00, 235/00)

(54) **2,3-Dihydroimidazo (1,2-c) pyrimidines, their preparation, formulations containing them and their use as pharmaceuticals.**

(30) Priority: **09.03.78 US 884884**

(43) Date of publication of application:
**19.09.79 Bulletin 79/19**

(45) Publication of the grant of the patent:
**22.09.82 Bulletin 82/38**

(84) Designated Contracting States:
**DE GB IT NL SE**

(56) References cited:
**JOURNAL OF THE CHEMICAL SOCIETY, (C), 1971, London**
**J. CLARK and T. RAMSDEN: "Heterocyclic Studies. Part XVII. Some 2,3-Dihydroimidazo-(1,2-a)- and (1,2-c)-pyrimidines", pages 679—83.**

(73) Proprietor: **ELI LILLY AND COMPANY**
**307, East McCarty Street**
**Indianapolis Indiana 46285 (US)**

(72) Inventor: **Turner, William Wilson**
**4821 N. College Avenue**
**Indianapolis Indiana (US)**

(74) Representative: **Crowther, Terence Roger**
**European Patent Attorney et al,**
**Erl Wood Manor**
**Windlesham Surrey GU20 6PH (GB)**

**EP 0 004 173 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 2,3-Dihydroimidazo[1,2-c]pyrimidines, their preparation, formulations containing them and their use as pharmaceuticals

This invention provides di-substituted 2,3-dihydroimidazo[1,2-c]pyrimidines which are useful as pharmaceuticals, in particular, as anti-viral and immunoregulative agents, a process for preparing them and pharmaceutical compositions making use of them.

The Ring Index, page 155 (second edition, American Chemical Society, 1960) gives the basic imidazo[1,2-c]pyrimidine structure with a reference to *Ann, 432,* 120 (1923). Substituted imidazo[1,2-c]-pyrimidines are also referred to in the more recent literature; for example, Bartholomew, et al. *J. Med. Chem., 19,* 814 (1976) gives the preparation of the arabinosyl hypoxanthine and arabinosyl guanine analogs of the imidazo[1,2-c]pyrimidine series. The intermediate compound 7-chloroimidazo[1,2-c]pyrimidine-5(6H)-one is also referred to. None of the compounds disclosed in this publication exhibited significant anti-viral or anti-microbial activity in vitro. West German patent 2511316 issued September 18, 1975, (Derwent Abstract 64314W/39, 1975) to Eisai KK discloses a group of 2-alkyl-5-alkylmercapto-7-hydroxyimidazo[1,2-c]pyrimidines. They are alleged to be useful as anti-inflammatory or analgesic agents.

A single reference to chloro-substituted 2,3-dihydro-imidazo[1,2-c]pyrimidines is available; see Yanai et al. *Yakugaku Zasshi, 94,* 1503, (1974). Compound XVII on page 1506 is 7-chloro-2,3-dihydroimidazo[1,2-c]pyrimidine hydrochloride. Both the hydrochloride salt and the free base are characterized in Tables I and Table V and analytical data are provided in Table VIII. The corresponding unhydrogenated compound, XXXII, is also disclosed. No utility is set forth therein for these compounds.

This invention provides substituted 2,3-dihydroimidazo[1,2-c]pyrimidines of the structure

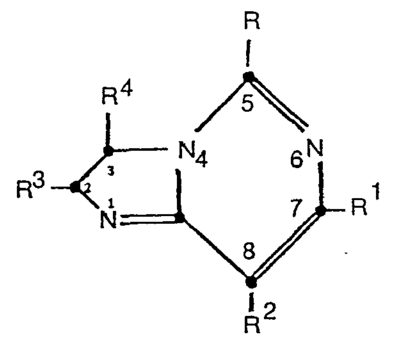

I

wherein R is $C_1$—$C_5$ alkyl, phenyl, amino or methylthio, $R^1$ is chloro or methylamino and $R^2$, $R^3$ and $R^4$ individually are hydrogen, methyl or phenyl, and pharmaceutically-acceptable acid addition salts thereof; and the compound 7-(2-chloroethylamino)-8-nitro-2,3-dihydroimidazo[1,2-c]pyrimidine and its pharmaceutically-acceptable acid addition salts.

The compounds of formula I above are prepared by reacting a compound of the formula

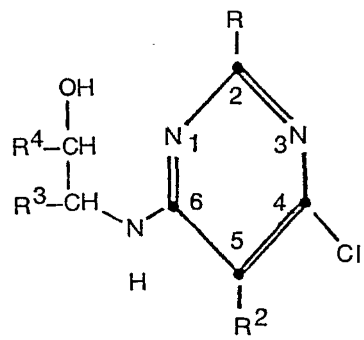

II

wherein R, $R^2$, $R^3$ and $R^4$ are as defined above, with thionyl chloride or trifluoromethylsulfonic acid, to obtain the compound of formula I wherein $R^1$ is chloro, and optionally reacting the compound so obtained with methylamine to obtain the compound of formula I wherein $R^1$ is methylamino and recovering the product in the free base form or as a pharmaceutically-acceptable acid addition salt; or reacting 4,6-dichlorol-5-nitropyrimidine with 2-chloroethylamine to obtain 7-(2-chloroethylamino)-8-nitro-2,3-dihydroimidazo[1,2-c]pyrimidine, and recovering the product in the free base form or as a pharmaceutically-acceptable acid addition salt.

The illustrative $C_1$—$C_5$ alkyl groups which R can represent include methyl, ethyl, n-propyl, isopropyl, n-butyl, isobutyl, sec-butyl, n-amyl, isoamyl, 1,2-dimethylpropyl, sec-amyl, t-amyl, and the like.

Compounds according to formula I in which $R^1$ is methylamino form pharmaceutically-acceptable acid addition salts derived from inorganic acids such as hydrochloric acid, nitric acid, phosphoric acid, sulfuric acid, hydrobromic acid, hydriodic acid, nitrous acid, phosphorous acid and the like; and organic acids including particularly the organic sulfonic acids. Pharmaceutically-acceptable salts of this invention thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, benzenesulfonate, toluenesulfonate, chlorobenzenesulfonate, xylenesulfonate, methanesulfonate, trifluoromethylsulfonate, propanesulfonate, naphthalene-1-sulfonate, naphthalene-2-sulfonate, acetate, oxalate and lactate.

Illustrative compounds coming within the scope of this invention include:

5,8-Dimethyl-7-chloro-2,3-dihydroimidazo[1,2-c]pyrimidine sulfate,
2,5-Dimethyl-7-chloro-2,3-dihydroimidazo[1,2-c]pyrimidine hydrobromide,
2-Phenyl-5-amino-7-chloro-2,3-dihydroimidazo[1,2-c]pyrimidine dihydrogenphosphate,
2,3,5-Trimethyl-7-chloro-2,3-dihydroimidazo[1,2-c]pyrimidine metaphosphate,
3-Phenyl-7-methylamino-2,3-dihydroimidazo[1,2-c]pyrimidine hydrochloride,
5-Isoamyl-7-methylamino-2,3-dihydroimidazo[1,2-c]pyrimidine hydrochloride.

The compounds of formula I wherein $R^1$ is chlorine are prepared by the following reaction scheme:

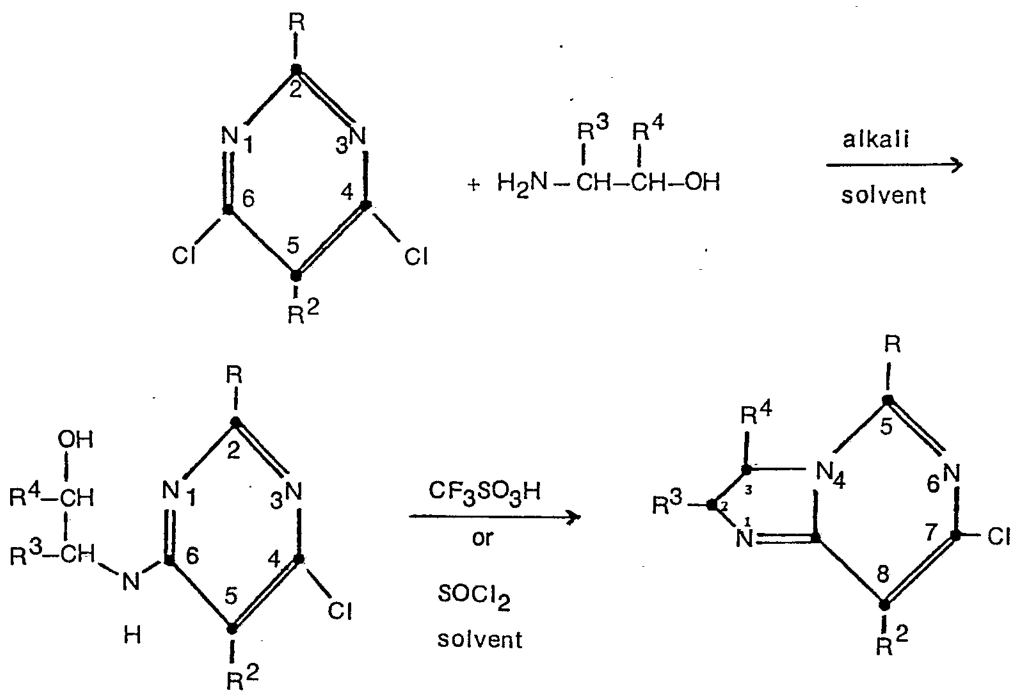


II I

wherein R, $R^2$, $R^3$ and $R^4$ have the same meaning as hereinabove.

According to the above reaction scheme, the starting compounds of formula II are prepared by reacting a 4,6-dichloropyrimidine substituted at C—2 with the desired R group and optionally substituted at C—5 with methyl or phenyl with an ethanol amine in an inert non-hydroxylic solvent in the presence of a suitable alkali. Useful solvents for this preparation include ethers such as tetrahydrofuran (THF), dioxane, and the like, and amides such as dimethylacetamide and dimethylformamide. Mild alkaline conditions should be employed, such as the presence of sodium bicarbonate, in order to avoid replacing the second chlorine atom in the pyrimidine by hydroxy. Ordinarily, equimolar quantities of ethanolamine and pyrimidine are employed. In addition, it is preferred to add the ethanolamine to the pyrimidine solution so as to minimize the production of diamino-substituted pyrimidine by-products.

The 4-chloro-6-(2-hydroxyethyl)aminopyrimidine of formula II thus produced is cyclized using trifluoromethylsulfonic acid or thionyl chloride to yield the dihydroimidazopyrimidine of formula I of this invention wherein $R^1$ is chloro. In carrying out the cyclization reaction, it is important that the solvent does not react under Friedel-Craft conditions with the hydroxylethyl group. Solvents include benzene, deactivated benzenes such as nitrobenzene and chlorobenzene, or cyclohexane, should be employed when the reagent is trifluoromethylsulfonic acid. Activated benzenes such as toluene, anisole and the like should be avoided as solvents. In cyclizing with thionyl chloride, a solvent such as THF, or other relatively high molecular weight ethers including dialkoxyalkanes such as bis(2-methoxyethyl)ether, is customarily employed. The cyclization reaction requires heating in the range 50°C. to 110°C.

The compounds of this invention wherein $R^1$ is methylamino are prepared by reacting a compound according to formula I above wherein $R^1$ is chloro with methylamine. This replacement reaction is carried out by simply mixing the amine and the 7-chloro-2,3-dihydroimidazo[1,2-c]pyrimidine in the form of an acid addition salt. Saturated sodium bicarbonate solution is a reaction medium for this reaction; alcohols including lower alkanols, especially ethanol, and amides, especially dimethylformamide and dimethylacetamide, are also useful. The reaction takes place well at ambient temperature; a temperature range from 0°C. to the reflux temperature may be used.

The compounds of this invention according to Formula I wherein $R^3$ or $R^4$ are other than hydrogen, contain an asymmetric carbon atom. For example, 2,5-dimethyl-7-chloro-2,3-dihydroimidazo[1,2-c]pyrimidine is produced in the above synthetic procedure as a *dl.* mixture or racemate, the asymmetric carbon giving rise to optical isomers, in this case at C—2. This *dl* mixture can be resolved into its optical antipodes by methods known to the art or, alternatively, an optically active ethanolamine can be used to prepare the starting compound of formula II.

When thionyl chloride is used as the reagent in the reaction with the compound of formula II, the product is the hydrochloride salt; when trifluoromethylsulfonic acid is used, the product is the trifluoromethylsulfonate. The initial products may be transformed into other salts by contacting them with ion exchange resins charged with the desired anion. When the above initial product is further reacted to form the compound wherein $R^1$ is methylamino, the product may be recovered in the form of any desired salt by further routine reaction of the product with the appropriate acid, as in aqueous solution, in an inert solvent such as an alcohol or ketone, or in an aqueous solvent. Salts of 7-(2-chloroethylamino)-8-nitro-2,3-dihydroimidazo[1,2-c]pyrimidine are prepared similarly.

This invention is further illustrated by the following specific examples:

Preparation 1

One hundred grams of 4,6-dichloro-2-methylpyrimidine and 37.40 g. of 2-aminoethanol were refluxed overnight in THF in the presence of an excess of sodium bicarbonate. The reaction mixture was filtered after cooling and the solvent removed in vacuo. The reaction product remaining as a residue was slurried in ether and the slurry filtered. A yield of 98 g. of 2-methyl-4-chloro-6-β-hydroxyethylaminopyrimidine was collected.

Example 1

5-Methyl-7-chloro-2,3-dihydroimidazo[1,2-c]pyrimidine hydrochloride

The above compound was dissolved in benzene without further purification and 238.5 g. of trifluoromethylsulfonic acid were added. The consequent 2-phase reaction system was heated to refluxing temperature overnight. After cooling, water was added to the reaction mixture and the aqueous phase separated. The aqueous phase was evaporated in vacuo, leaving a residual oil comprising 5-methyl-7-chloro-2,3-dihydroimidazo[1,2-c]pyrimidine formed in the above reaction as the trifluoromethylsulfonic salt; weight = 92 g. The salt was crystallized from an ether-ethyl acetate solvent mixture. The crystalline mixture was dissolved in water and treated with the chloride form of an ion exchange resin, thus forming 5-methyl-7-chloro-2,3-dihydroimidazo[1,2-c]pyrimidine hydrochloride. The compound melted about 230°C. after recrystallization from an ethanol-ethyl acetate solvent mixture; yield = 17 g.

| | | | |
|---|---|---|---|
| Analysis Calc.: | C, 40.80; | H, 4.40; | N, 20.39; |
| Found: | C, 41.05; | H, 4.44; | N, 20.67. |

Example 2

5-Phenyl-7-chloro-2,3-dihydroimidazo[1,2-c]pyrimidine hydrochloride

A 6.2 g. portion of 4-chloro-6-(2-hydroxyethylamino)-2-phenylpyrimidine was reacted with 5.8 ml of trifluoromethylsulfonic acid according to the process of example 1 to obtain 1.3 g. of 5-phenyl-7-chloro-2,3-dihydroimidazo[1,2-c]pyrimidine hydrochloride, m.p. 230°C., decomposition.

Example 3

*dl*-3,5-Dimethyl-7-chloro-2,3-dihydroimidazo[1,2-c]pyrimidine hydrochloride

According to the process of example 1, 5 g. of 4-chloro-6-(2-hydroxypropylamino)-2-methylpyrimidine was reacted with 2.3 ml of trifluoromethylsulfonic acid to obtain 0.37 g. of *dl*-3,5-dimethyl-7-chloro-2,3-dihydroimidazo[1,2-c]pyrimidine hydrochloride, m.p. 150°C.

Example 4

*dl*-3-Phenyl-5-methyl-7-chloro-2,3-dihydroimidazo[1,2-c]pyrimidine hydrochloride

A 5 g. portion of 4-chloro-6-(2-hydroxy-2-phenylethylamino)-2-methylpyrimidine was reacted with 1.75 ml. of trifluoromethylsulfonic acid following the general procedure of example 1 to obtain 0.95 g. of *dl*-3-phenyl-5-methyl-7-chloro-2,3-dihydroimidazo[1,2-c]pyrimidine hydrochloride, initial m.p. 180°C., decomp.

Example 5

*dl*-3-Phenyl-5-methylthio-7-chloro-2,3-dihydroimidazo[1,2-c]pyrimidine hydrochloride.

The process of example 1 was followed in general in the reaction of 2.4 g. of 4-chloro-6-(2-hydroxy-2-phenylethylamino)-2-methylthiopyrimidine with 2.14 ml. of trifluoromethylsulfonic acid to obtain 0.54 g. of *dl*-3-phenyl-5-methylthio-7-chloro-2,3-dihydroimidazo[1,2-c]pyrimidine hydrochloride, initial m.p. 200°C., decomp.

Example 6

2-Phenyl-5-methyl-7-chloro-2,3-dihydroimidazo[1,2-c]pyrimidine hydrochloride.

Seven g. of 4-chloro-6-(2-hydroxy-1-phenylethylamino)-2-methylpyrimidine was reacted with 5 ml of trifluoromethylsulfonic acid according to the process of example 1 to obtain 0.43 g. of 2-phenyl-5-methyl-7-chloro-2,3-dihydroimidazo[1,2-c]pyrimidine hydrochloride, m.p. 150°C., decomp.

Example 7

5-Methylthio-7-chloro-2,3-dihydroimidazo[1,2-c]pyrimidine hydrochloride.

A 15 g. portion of 4-chloro-6-(2-hydroxyethylamino)-2-methylthiopyrimidine was dissolved in 300 ml of tetrahydrofuran, and 16.7 g. of thionyl chloride was added dropwise. The mixture was then stirred at the reflux temperature for 4 hours, and the solids were collected by filtration. The solids were then dissolved in water, and the water was evaporated under vacuum. The product was then crystallized from ethanol/ethyl acetate to obtain 13 g. of 5-methylthio-7-chloro-2,3-dihydroimidazol-[1,2-c]pyrimidine hydrochloride, initial m.p. 220°C., decomp.

Example 8

5-Ethyl-7-chloro-2,3-dihydroimidazo[1,2-c]pyrimidine hydrochloride.

A 31.5 g. portion of 4-chloro-2-ethyl-6-(2-hydroxyethylamino)pyrimidine was reacted with 84 ml. of thionyl chloride according to the general process of example 7 to produce 6.6 g. of 5-ethyl-7-chloro-2,3-dihydroimidazo[1,2-c]pyrimidine hydrochloride, initial m.p. 220°C., decomp.

Example 9

5-Amino-7-chloro-2,3-dihydroimidazo[1,2-c]pyrimidine hydrochloride.

Following the general process of example 7, 7.5 g. of 2-amino-4-chloro-6-(2-hydroxyethylamino)-pyrimidine was reacted with 10 ml. of thionyl chloride to obtain 1.1 g. of 5-amino-7-chloro-2,3-dihydroimidazo[1,2-c]pyrimidine hydrochloride, initial m.p. 260°C., decomp.

Example 10

5-n-Amyl-7-chloro-2,3-dihydroimidazo[1,2-c]pyrimidine hydrochloride.

A 28 g. portion of 2-n-amyl-4-chloro-6-(2-hydroxyethylamino)pyrimidine was reacted according to the process of example 7 with 84 ml. of thionyl chloride to obtain 0.33 g. of 5-n-amyl-7-chloro-2,3-dihydroimidazo[1,2-c]pyrimidine hydrochloride, initial m.p. 170°C.

Example 11

5-Methylthio-7-chloro-8-phenyl-2,3-dihydroimidazo[1,2-c]pyrimidine hydrochloride.

The process of example 7 was followed, in general, in reacting 15 g. of 4-chloro-6-(2-hydroxyethylamino)-2-methylthio-5-phenylpyrimidine with 12.1 g. of thionyl chloride to obtain 7.3 g of 5-methylthio-7-chloro-8-phenyl-2,3-dihydroimidazo[1,2-c]pyrimidine hydrochloride, initial m.p. 212°C., decomp.

Example 12

5-Methyl-7-methylamino-2,3-dihydroimidazo[1,2-c]pyrimidine dihydrochloride.

Two g. of 5-methyl-7-chloro-2,3-dihydroimidazo[1,2-c]pyrimidine hydrochloride was dissolved in ethanol at ambient temperature, and the solution was saturated with methylamine by bubbling methylamine gas through it. The solution was allowed to stand at ambient temperature for three hours, and the solvent was then removed under vacuum. The residue was taken up in ethanol, dilute hydrochloric acid was added, and the volatile portions were removed under vacuum. The residue was crystallized from ethanol/ethyl acetate to obtain 0.35 g. of 5-methyl-7-methylamino-2,3-dihydroimidazo[1,2-c]pyrimidine dihydrochloride, m.p. 250—253°C.

Example 13

7-(2-Chloroethylamino)-8-nitro-2,3-dihydroimidazo[1,2-c]pyrimidine.

A solution of 20 g. of 4,6-dichloro-5-nitropyrimidine was prepared in ether. An aqueous solution containing 14 g. of 2-chloroethylamine hydrochloride and 24 g. of sodium bicarbonate were added thereto and the subsequent mixture agitated for about 30 minutes. The ether layer was separated, and the separated layer washed with water and dried. Removal of the ether in vacuo yielded an oil containing chiefly 4-(2-chloroethylamino)-5-nitro-6-chloropyrimidine plus a small quantity of 5-nitro-4,6-bis(2-chloroethyl)pyrimidine (the disubstituted product) and a very small quantity of starting

material. The oily residue was allowed to stand in chloroform for about one day. A solid comprising 7-(2-chloroethylamino)-8-nitro-2,3-dihydroimidazo[1,2-c]pyrimidine hydrochloride precipitated and the precipitate was separated by filtration. 7-(2-Chloroethylamino)-8-nitro-2,3-dihydroimidazo[1,2-c]pyrimidine hydrochloride thus prepared decomposed at 135°C. after recrystallization from chloroform; yield = 5.378 g.

| | | | | |
|---|---|---|---|---|
| Analysis Calc.: | C, 34.30; | H, 3.96; | N, 25.00; | Cl, 25.31 |
| Found: | C, 34.42; | H, 3.82; | N, 25.08; | Cl, 25.59 |

The compounds of this invention are anti-viral agents and have demonstrated their activity against cutaneous *Herpes simplex* virus type I (HSU—1) in guinea pigs according to the following tests. Three epilated areas on each guinea pig back were inoculated with approximately 1 × $10^5$ plaque forming units of virus using a sterile vaccinating needle. Untreated guinea pigs so inoculated developed consistent rosette lesions in about 96 hours. All three areas on the back of each guinea pig were drug-treated and these compared with separate control animals which were treated with the particular vehicle employed for administering the drug. The positive control animals were treated with 0.5—1.0 percent phosphonoacetic acid (PAA) suspended in the same vehicle as the drug. Animals were inoculated on the morning of day 1 and treated on the afternoon of day 1. Two treatments per day were administered through day 5 for a total of 10 treatments. Hair was again epilated on day 5 and readings were begun then and taken daily through day 10. Lesions for each area were scored from 0, indicating no injury, to 4+, indicating a fully developed herpatic lesion with inflammation vesicle and pustules. An average score was then calculated for each drug for each day for the number of treated areas and control areas. Usually 3—6 guinea pigs were used per drug. After scoring, a final score or mean of means was calculated for drug-treated and for control areas. Table I which follows gives the results of determinations carried out as outlined above on compounds coming within the scope of this invention. In the table, column 1 gives the name of the drugs, column 2, the vehicle, column 3, the concentration; column 4, the number of animals used; and column 5, the final score.

TABLE I

Topical Treatment

| Name of Compound | Vehicle* | % Conc. | No. Animals | Final Score |
|---|---|---|---|---|
| 5-Methylthio-7-chloro-2,3-dihydroimidazo[1,2-c]pyrimidine hydrochloride | R | 2.5 | 4 | 2.00 |
| 5-Methylthio-7-chloro-8-phenyl-2,3-dihydroimidazo[1,2-c]-pyrimidine hydrochloride | D | 2.5 | 4 | 1.97 |
| 5-Methyl-7-chloro-2,3-dihydro-imidazo[1,2-c]pyrimidine hydro-chloride | K | 1.0 | 4 | 1.95 |
| | D | 1.0 | 4 | 1.56 |
| | B | 1.0 | 4 | 2.06 |
| | R | 1.0 | 4 | 1.81 |
| 3-Phenyl-5-methyl-7-chloro-2,3-dihydroimidazo[1,2-c]-pyrimidine hydrochloride | D | 2.5 | 4 | 1.72 |
| 5-Ethyl-7-chloro-2,3-dihydro-imidazo[1,2-c]pyrimidine hydrochloride | D | 2.5 | 3 | 1.60 |
| | D | 1.0 | 4 | 1.09 |
| | D | 1.5 | 4 | 1.53 |

A second in-vivo test against *Herpes simplex* virus type I and *Herpes simplex* virus type II was carried out as follows. Guinea pigs were inoculated intravaginally by swabbing the vagina with an absorbent cotton swab containing 2 × $10^4$ plaque forming units of the virus. Prior to the inoculation

with the virus, the vagina was swabbed with physiological saline to remove potential virus inhibitors. Treatment was started four hours after inoculation and continued for four days. The drug in a suitable base was introduced into the vaginal area also with an absorbent cotton swab. Controls in which the vehicle only were swabbed were included. Beginning on day 5 after inoculation and continuing through day 10, each animal was examined and scored from 0 to 4+ for secretion, inflammation, vesiculation and necrosis, thus yielding a possible top score of +16 per guinea pig. The results of this determination are included in Table 2. In the table, column 1 gives the name of the compound; column 2, the vehicle; column 3, the concentration; column 4, the number of animals used; and columns 5, the final score.

TABLE 2

Intravaginal Treatment

| Name of Compound | Vehicle* | % Conc. | No. Animals | Final Score |
|---|---|---|---|---|
| 7-(2-chloroethylamino)-8-nitro-2,3-dihydroimidazo-[1,2-c]pyrimidine hydrochloride | R | 2.5 | 4 | 1.25 |
| 5-Methylthio-7-chloro-2,3-dihydroimidazo[1,2-c]-pyrimidine hydrochloride | R | 2.5 | 4 | .92 |
| | D | 2.5 | 4 | 3.81 |
| 5-Methyl-7-chloro-2,3-dihydroimidazo[1,2-c]-pyrimidine hydrochloride | C | 2.5 | 5 | 7.9 |
| | D | 2.0 | 5 | 6.13 |
| | R | 2.0 | 4 | 3.17 |
| | R | 2.0 | 5 | 3.57 |
| 5-Ethyl-7-chloro-2,3-dihydroimidazo[1,2-c]pyrimidine hydrochloride | R | 2.5 | 4 | 2.83 |

The compounds of this invention also have anti-viral activity in vitro against *Herpes simplex* virus. In order to demonstrate this activity, the following test method was employed.

African green monkey kidney cells (BSC—1) or Hela cells (5—3) were grown in 25 cc. Falcon flasks at 37°C. in medium 199 with 5 percent inactivated fetal bovine serum (FBS), penicillin (150 units/ml.) and streptomycin (150 mcg./ml.). When confluent monolayers were formed, the supernatant growth medium was removed and 0.3 ml. of an appropriate dilution of *Herpes simplex* virus was added to each flask. After adsorption for one hour at room temperature, the virus infected cell sheet was overlaid with a medium comprising one part of 1 percent Ionagar No. 2 and one part double strength medium 199 with FCS, (fetal calf serum), penicillin, and streptomycin and also containing drug at concentrations of 100, 50, 25, 12, 6 and 3 micrograms per milliliter (mcg./ml.). The flask containing no drug served as a control. The stock solutions of 2,3-dihydroimidazo[1,2-c]pyrimidine compounds were made up in dimethylsulfoxide dilution at a concentration of $10^4$ mcg./ml. The flasks were incubated for 72 hours at 37°C. Plaques were seen in those areas where the virus infected and reproduced in the cells. A solution of 10 percent formalin and 2 percent sodium acetate was added to each flask to inactivate the virus and fix the cell sheet to the surface of the flask. The virus plaques, irrespective of size, were counted after staining the surrounding cell areas with crystal violet. The plaque count was compared to the control count at each drug concentration. The activity of the test compound was expressed as percentage plaque inhibition. Table 3, which follows, embodies the results of these determinations. In the table, column 1 gives the name of the compound, columns 2 through 6, the percent inhibition of *Herpes simplex* plaques at various mcg./ml. levels of drug.

TABLE 3

| Name of compound | PERCENT INHIBITION OF PLAQUES AT | | | | | |
|---|---|---|---|---|---|---|
| | 100 mcg/ml | 50 mcg/ml | 25 mcg/ml | 12 mcg/ml | 6 mcg/ml | 3 mcg/ml |
| 5-Methyl-7-chloro-2,3-dihydro-imidazo[1,2-c]pyrimidine hydrochloride | T<br>T | 85<br>82 | 0<br>11 | 0 | 0 | |
| 5-n-Amyl-7-chloro-2,3-dihydro-imidazo[1,2-c]pyrimidine hydrochloride | T<br>T | 100 (ST)<br>100 (ST) | 29<br>87 | 0<br>41 | 0<br>0 | 0 |
| 5-Ethyl-7-chloro-2,3-dihydro-imidazo[1,2-c]pyrimidine hydrochloride | T<br>100 (ST) | 100<br>76 | 50<br>33 | 10<br>30 | 0<br>33 | 0<br>0 |
| 5-Amino-7-chloro-2,3-dihydro-imidazo[1,2-c]pyrimidine hydrochloride | 11 | 0 | 0 | 0 | 0 | 0 |
| 5-methyl-7-methylamino-2,3-dihydroimidazo[1,2-c]-pyrimidine dihydrochloride | 30 | 18 | 27 | 0 | 0 | |
| 5-Methylthio-7-chloro-2,3-dihydroimidazo[1,2-c]-pyrimidine hydrochloride | T<br>T | T<br>T | T<br>T | 100<br>T | 49<br>48 | 30 |

TABLE 3 (Continued)

| Name of Compound | PERCENT INHIBITION OF PLAQUES AT 100 mcg/ml | 50 mcg/ml | 25 mcg/ml | 12 mcg/ml | 6 mcg/ml | 3 mcg/ml |
|---|---|---|---|---|---|---|
| 5-Methylthio-7-chloro-8-phenyl-2,3-dihydroimidazo[1,2-c]-pyrimidine hyrochloride | T<br>T | T<br>T | T<br>T | T<br>T | T<br>T | 100 (ST)<br>58 |
| (±) 3-Phenyl-5-methyl-7-chloro-2,3-dihydro-imidazo[1,2-c]pyrimidine hydrochloride | T<br>T | 100 (ST)<br>95 | 78<br>68 | 4<br>23 | 0<br>0 | 0<br>0 |
| (±) 2-Phenyl-5-methyl-7-chloro-2,3-dihydro-imidazo[1,2-c]pyrimidine hydrochloride | T<br>T | 100 (ST)<br>89 | 87<br>83 | 41<br>50 | 0<br>13 | 0<br>0 |
| (±) 3,5-Dimethyl-7-chloro-2,3-dihydroimidazo[1,2-c]-pyrimidine hydrochloride | T | 74 | 12 | 0 | 0 | |
| (±) 3-Phenyl-5-methylthio-7-chloro-2,3-dihydro-imidazo[1,2-c]pyrimidine hydrochloride | T (LV)<br>T (HV) | T<br>T | T<br>T | T<br>T | 100<br>84 | 57<br>23 |
| 7-(2-chloroethylamino)-8-nitro-2,3-dihydroimidazo[1,2-c]-pyrimidine hydrochloride | MT | MT | 68 (MT) | 41 | 33 | |

MT — moderately toxic
T — toxic
ST — slightly toxic
LV — low virus input
HV — high virus

In accordance with the data presented in the above table, those compounds according to Formula I in which $R^1$ is chloro, form a preferred class of the compounds of this invention and more particularly, those compounds according to Formula I in which $R^1$ is chloro, and R is $C_1$—$C_8$ alkyl or methylmercapto constitute a particularly preferred group of compounds.

Compounds coming within the scope of Formula I above are able to suppress the growth of *Herpes simplex* virus on various surfaces wherein said virus is multiplying. These surfaces include non-living surfaces such as hospital glassware, hospital working surfaces and the like. The compounds can also be administered to mammals topically both to skin surfaces and particularly to mucosal membranes such as those present in the oral cavities and in the vagina. For such topical application, vehicles such as those listed in Table 4 below may be employed. A particularly useful vehicle for application to mucosal membranes is vehicle R, a cream base.

TABLE 4

Vehicles From Tables 1 & 2

B = Water

C = Cream composed of:

| | (Unit Formula) |
|---|---|
| Isopropyl Myristate | 3% |
| Polyethylene ether of stearyl alcohol (Polawax) | 8% |
| Squalene | 3% |
| Beeswax (White) | 1.5% |
| Glycerine | 5% |
| Preservatives | |
| Purified Water q.s. to | 100% |

D = Merthiolate Cream composed of: (Unit Formula)

1 part Thimerosal (optional)
150 parts Stearic Acid
25 parts Cetyl Alcohol
40 parts Mineral Oil
100 parts Glycerin
125 parts Triethanolamine
10 parts Polyoxyethylene Sorbitan Monostearate
Purified Water q.s. to 1000 parts

R = Cream base without Merthiolate

The particular pharmaceutical vehicle or carrier for the antiviral agents of formula I should be non-irritating either to skin or mucosa. Obviously, in general, skin can tolerate a greater degree of irritation in a carrier than can mucosa, but care should be taken in selection of a carrier that it be non-irritating for the particular body surface, internal or external, to which it is to be applied.

For treatment of *Herpes simplex* infections of the skin or mucosal surfaces, a compound of this invention is applied to the infected area as a 0.5—5.0% solution or suspension of an antiviral drug in salt form according to formula I above. The concentration of drug should be such that an anti-*Herpes*

quantity can readily be delivered by application to the infected site of a thin layer of drug-containing vehicle, whether it be a cream base, a jelly, or a high-viscosity aqueous vehicle.

Pharmaceutical compositions of this invention comprise a compound of formula I in a pharmaceutically-acceptable carrier adapted for topical use. The vehicles described above are typical of such carriers.

**Claims**

1. A compound of the formula

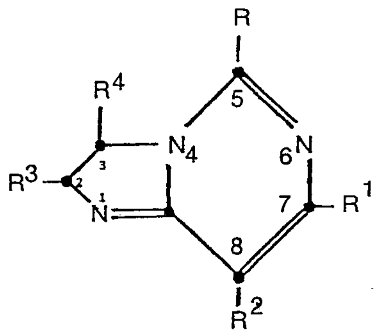

I

wherein R is $C_1$—$C_5$ alkyl, phenyl, amino or methylthio, $R^1$ is chloro or methylamino and $R^2$, $R^3$ and $R^4$ individually are hydrogen, methyl or phenyl, and pharmaceutically-acceptable acid addition salts thereof.

2. The compound of claim 1 which is 5-mēthyl-7-chloro-2,3-dihydroimidazo[1,2-c]pyrimidine and its pharmaceutically-acceptable salts.

3. The compound of claim 1 which is 5-methylthio-7-chloro-2,3-dihydroimidazo[1,2-c]pyrimidine and its pharmaceutically-acceptable salts.

4. The compound of claim 1 which is 5-methylthio-7-chloro-8-phenyl-2,3-dihydroimidazo[1,2-c]-pyrimidine and its pharmaceutically-acceptable salts.

5. The compound of claim 1 which is *dl*-3-phenyl-5-methyl-7-chloro-2,3-dihydroimidazo[1,2-c]pyrimidine and its pharmaceutically-acceptable salts.

6. The compound of claim 1, which is 5-ethyl-7-chloro-2,3-dihydroimidazo[1,2-c]pyrimidine and its pharmaceutically-acceptable salts.

7. 7-(2-Chloroethylamino)-8-nitro-2,3-dihydroimidazo[1,2-c]pyrimidine and its pharmaceutically-acceptable salts.

8. A pharmaceutical formulation comprising a compound as defined in any of claims 1—7 and a pharmaceutically-acceptable carrier suitable for topical application.

9. A compound as defined in any of claims 1—7 for inhibiting infections caused by *Herpes simplex* virus.

10. A process for preparing a compound of formula I as defined in any of claims 1—6 comprising reacting a compound of the formula

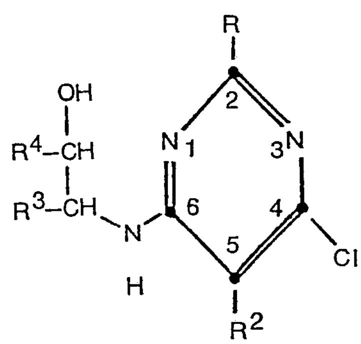

II

wherein R, $R^2$, $R^3$ and $R^4$ are as defined above, with thionyl chloride or trifluoromethylsulfonic acid, to obtain the compound of formula I wherein $R^1$ is chloro, and optionally reacting the compound so obtained with methylamine to obtain the compound of formula I wherein $R^1$ is methylamino and recovering the product in the free base form or as a pharmaceutically-acceptable acid addition salt.

11. A compound as defined in any of claims 1—7 for use as a pharmaceutical.

**Revendications**

1. Composé de la formule:

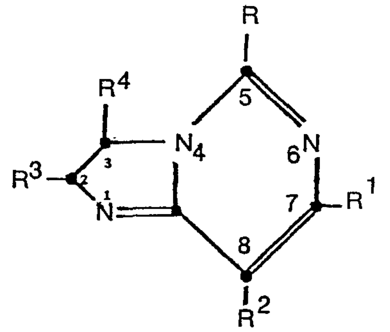

I

dans laquelle R est un groupe alcoyle en $C_1$—$C_5$, phényle, amino ou méthylthio, $R^1$ est un substituant chloro ou méthylamino et $R^2$, $R^3$ et $R^4$ sont chacun individuellement de l'hydrogène ou un groupe méthyle ou phényle, et ses sels d'addition d'acide pharmaceutiquement acceptables.

2. Composé selon la revendication 1, caractérisé en ce que c'est la 5-méthyl-7-chloro-2,3-dihydroimidazo[1,2-c]pyrimidine ou un sel pharmaceutiquement acceptable de ce composé.

3. Composé selon la revendication 1, caractérisé en ce que c'est la 5-méthylthio-7-chloro-2,3-dihydroimidazo[1,2-c]pyrimidine ou un sel pharmaceutiquement acceptable de ce composé.

4. Composé selon la revendication 1, caractérisé en ce que c'est la 5-méthylthio-7-chloro-8-phényl-2,3-dihydroimidazo[1,2-c]pyrimidine ou un sel pharmaceutiquement acceptable de ce composé.

5. Composé selon la revendication 1, caractérisé en ce que c'est la *dl*-3-phényl-5-méthyl-7-chloro-2,3-dihydroimidazo[1,2-c]pyrimidine ou un sel pharmaceutiquement acceptable de ce composé.

6. Composé selon la revendication 1, caractérisé en ce que c'est la 5-éthyl-7-chloro-2,3-dihydroimidazo[1,2-c]pyrimidine ou un sel pharmaceutiquement de ce composé.

7. 7-(2-chloroéthylamino)-8-nitro-2,3-dihydroimidazo[1,2-c]pyrimidine et ses sels pharmaceutiquement acceptables.

8. Composition pharmaceutique caractérisée en ce qu'elle comprend un composé tel que défini dans l'une quelconque des revendications 1 à 7 et un véhicule pharmaceutiquement acceptable convenable pour application topique.

9. Utilisation d'un composé tel que défini dans l'une quelconque des revendications 1 à 7 pour inhiber des infections causées par le virus *Herpes simplex.*

10. Procédé de préparation d'un composé de formule I tel que défini dans l'une quelconque des revendications 1 à 6, caractérisé en ce que fait réagir un composé de la formule:

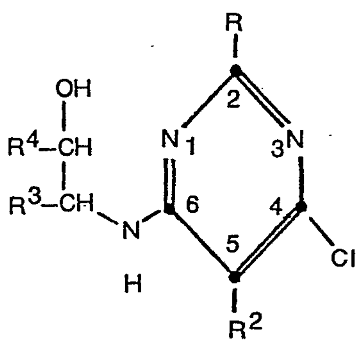

II

dans laquelle R, $R^2$, $R^3$ et $R^4$ sont tels que définis ci-dessus, avec le chlorure de thionyle ou l'acide trifluorométhylsulfonique, pour obtenir le composé de formule I dans lequel $R^1$ est un substituant chloro, et éventuellement on fait réagir le composé ainsi obtenu avec la méthylamine pour obtenir le composé de formule I dans lequel $R^1$ est un substituant méthylamino et on recueille le produit sous la forme de la base libre ou d'un sel d'addition d'acide pharmaceutiquement acceptable.

11. Utilisation comme produit pharmaceutiquement d'un composé tel que défini dans l'une quelconque des revendications 1 à 7.

## Patentansprüche

1. 2,3-Dihydroimidazo[1,2-c]pyrimidine der allgemeinen Formel I

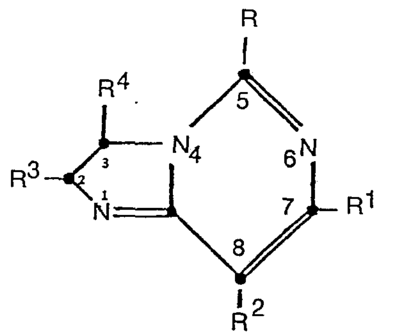

I

worin R für $C_1$—$C_5$-Alkyl, Phenyl, Amino oder Methylthio steht,
$R^1$ Chlor oder Methylamino bedeutet und
$R^2$, $R^3$ sowie $R^4$ unabhängig voneinander Wasserstoff, Methyl oder Phenyl darstellen, und die pharmazeutisch unbedenklichen Säureadditionssalze hiervon.

2. 5-Methyl-7-chlor-2,3-dihydroimidazo[1,2-c]pyrimidin und dessen pharmazeutisch unbedenkliche Salze nach Anspruch 1.

3. 5-Methylthio-7-chlor-2,3-dihydroimidazo[1,2-c]pyrimidin und dessen pharmazeutisch unbedenkliche Salze nach Anspruch 1.

4. 5-Methylthio-7-chlor-8-phenyl-2,3-dihydroimidazo[1,2-c]pyrimidin und dessen pharmazeutisch unbedenkliche Salze nach Anspruch 1.

5. dl-3-Phenyl-5-methyl-7-chlor-2,3-dihydroimidazo[1,2-c]pyrimidin und dessen pharmazeutisch unbedenkliche Salze nach Anspruch 1.

6. 5-Ethyl-7-chlor-2,3-dihydroimidazo[1,2-c]pyrimidin und dessen pharmazeutisch unbedenkliche Salze nach Anspruch 1.

7. 7-(2-Chloroethylamino)-8-nitro-2,3-dihydroimidazo[1,2-c]pyrimidin und dessen pharmazeutisch unbedenkliche Salze.

8. Pharmazeutische Formulierung, dadurch gekennzeichnet, daß sie eine Verbindung nach einem der Ansprüche 1 bis 7 und einen für eine topische Anwendung geeigneten pharmazeutisch unbedenklichen Träger enthalt.

9. Verwendung einer Verbindung nach einem der Ansprüche 1 bis 7 zur Hemmung von durch den Virus Herpes simplex hervorgerufenen Infektionen.

10. Verfahren zur Herstellung eines 2,3-Dihydroimidazo[1,2-c]pyrimidins der allgemeinen Formel I gemäß einem der Ansprüche 1 bis 6, dadurch gekennzeichnet, daß man eine Verbindung der allgemeinen Formel II

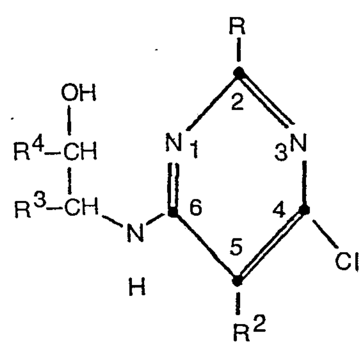

II

worin R, $R^2$, $R^3$ und $R^4$ die oben angegebenen Bedeutungen haben, durch Umsetzung mit Thionylchlorid oder Trifluormethylsulfonsäure in eine Verbindung der allgemeinen Formel I überführt, worin $R^1$ für Chlor steht, und die so erhaltene Verbindung gegebenenfalls durch Umsetzung mit Methylamin in eine Verbindung der allgemeinen Formel I überführt, worin $R^1$ Methylamino bedeutet, und das gewünschte Produkt in Form der freien Base oder eines pharmazeutisch unbedenklichen Säureadditionssalzes gewinnt.

11. Verwendung einer Verbindung nach einem der Ansprüche 1 bis 7 als Pharmazeutikum.